# EUROPEAN PATENT SPECIFICATION

(11) **EP 2 517 570 B1**
(45) Date of publication and mention of the grant of the patent: **05.11.2014**
(21) Application number: 12172547.7
(22) Date of filing: 02.12.2010
(51) Int. Cl.: A23K 1/16, A23K 1/18

(54) **Methods and feed compositions for masking of fish semiochemicals**
Verfahren und Futterzusammensetzungen zur Maskierung von Fischbotenstoffen
Procédés et compositions d'alimentation pour masquer des produits sémiochimiques de poisson

(30) Priority: 02.12.2009 NO 20093460
(43) Date of publication of application: 31.10.2012
(62) Divisional of application: 10803171.7
(73) Proprietor: Ewos Innovation AS, 4335 Dirdal (NO)
(72) Inventor: Wadsworth, Simon, 9405 Harstad (NO); Vecino, Dr. José Luis González, 4326 Sandnes (NO)
(74) Representative: Acapo AS

(56) References cited:
- EP-A1- 1 208 751
- WO-A2-2004/091307
- GB-A- 2 388 544
- US-A- 3 662 069
- US-A- 5 504 081
- ROTH M ET AL: "Current practices in the chemotherapeutic control of sea lice infestations in aquaculture: A review", JOURNAL OF FISH DISEASES, OXFORD, GB, vol. 16, no. 1, 1 January 1993 (1993-01-01), pages 1-26, XP009146063, ISSN: 0140-7775

## Description

### FIELD OF THE INVENTION

The present invention relates to a method for masking the odor of fish semiochemicals in water. The invention also relates to a method for reducing the attraction between a parasite and a fish. The invention also relates to fish feed compositions, and the use of a compound for the prevention and/or treatment of a parasite infection in fish.

Currently available strategies are disclosed in e.g., Roth et al, Journal of Fish Diseases 1993; 16: 1-26; US 5 504 081; and EP 1 208 751.

### BACKGROUND TO THE INVENTION

Sea lice *(Lepeophtheirus salmonis, Caligus* sp.) are the major pathogen currently affecting the global salmon farming industry and have a significant impact on many areas of production. Economic impact on the aquaculture industry are high due to high annual losses. There is also continued concern over the impact of salmon farming on wild salmon populations with increased density of sea lice adjacent to these production sites. Control measures have been reliant upon the use of a number of chemotherapeutants since the 1970's. Reduced efficacy has now been reported for all compounds, with the exception of the insect growth regulators (IGR) diflubenzuron and teflubenzuron. Further methods are therefore required to effectively control sea lice, in conjunction with sea lice medicines.

### Host-specific parasites

The *Lepeophtheirus* genus of sea lice is a host-specific parasite. *L. salmonis* will only complete its life cycle on salmonid species, although mobile stages may occasionally be observed as opportunists on additional fish types. Other *Lepeophtheirus* sp. will target a narrow range of other fish species.

### Immune suppression of the host

The *Lepeophtheirus* genus of sea lice has evolved a range of mechanisms to suppress the immune response of their particular hosts. To overcome a potentially fatal inflammatory reaction the sea lice release a series of secretary / excretory products (SEP) into the host tissue, via salivary glands. Prostaglandins (PGE₂), alkaline phosphatase and a range of trypsin-like proteases have been identified as sea lice SEPs. It is thought that several additional unidentified factors such as phosphatase, apyrase and macrophage inhibition factor are also present.

### Effect of immune suppressants

*L. salmonis* has a significant immunosuppressive effect on a range of responses in Atlantic salmon including reduced respiratory burst, lower macrophage activity, increased apoptosis, necrosis, decreased numbers of mucosal cells and down-regulation of immune genes such as interleukin IL-1β and MHC-1. Suppression occurs at localised attachment sites, although a more generalised effect may occur with higher levels of sea lice infection. Once they have suppressed the immune system of the host, the lice are able to extend a frontal filament for a secure attachment. This is intimately associated with the host tissues and able to survive any subsequent immune response from that species.

### A fatal risk of attaching to the wrong host

*Lepeophtheirus* sp. are not able to suppress the immune system of non-host species. If lice try and settle on to a resistance fish species the immune response will kill it. Thus correct identification of the host is essential for attachment and survival of *Lepeophtheirus* sp.

### Correct host identification

Sea lice have advanced olfactory and contact chemoreceptors that are capable of accurate identification of specific host molecules. Semiochemicals (behaviour-modifying chemicals) are used by a range of arthropods in chemical communication systems to locate a host, mate or oviposition site. Similarly, many copepods use chemical cues to identify and seek out mates.

### Caligus species

Lice within the *Caligus* genus have an extensive range of potential hosts; *C elongatus* is known to infect over 80 host species world wide. *Caligus* have been found to posses a greater range and quantity of serine and non-serine proteases than *L. salmonis* and this may assist in defeating a greater range of immune responses from many different species. In addition *Caligus* deploy a different attachment mechanism that is not as intimately associated with host tissue. *Caligus* remove the epidermal tissue from the scales and then the frontal filament attaches directly to the cleared scales via a basal plate. The frontal filament is much longer than that deployed by *L. salmonis* and this allows the louse to remain at some distance from the host immune system. Despite these generalists adaption's some *Caligus* species still demonstrate a high degree of host specificity. This may develop in populations in areas where a particular host population is abundant such as *Caligus rogercresseyi* which are now the dominant sea lice species on salmon farms in Chile.

Through behavioral trials, tested the hypothesis that the inter-and intraspecific relationships of salmon louse, *C. rogercresseyi* are mediated by semiochemical compounds has been tested. It has been shown that the host species studied, Coho salmon, Atlantic salmon, and Rainbow trout, emit chemical signals that attract sea lice.

The object of the present invention is to provide a feed composition and a compound for prevention and control of sea lice attraction to, and infections in fish, preferable Salmonidae that is easily applicable, effective in long-term use and are considered as environmentally friendly and less toxic than many known chemotherapeutants. In particular, an object of the present invention is to provide a feed composition and a compound for masking the semiochemical compounds in order to reduce the attraction of a sea lice for salmonidae.

### SUMMARY OF THE INVENTION

A first aspect of the present invention relates to a compound for use in masking the odor of a fish semiochemical in water in reducing the attraction between a parasite and a fish, chararacterized in that a compound is added to said water or is administered to a fish in said water, wherein said compound is a compound of formula (II);

R¹-N=C=S (II)

wherein R¹ is C₁-C₄ alkyl or C₂-C₃ alkenyl or C₂-C₃-alkynyl or phenyl alkyl.

Preferably, said fish semiochemical is isophorone.

Preferably, said fish semiochemical is 1-Octen-3-ol or 6-methyl-5-hepten-2-one.

Preferably, said fish is a Salmonidae.

Preferably, said water is Salmonidae conditioned sea water or said fish in the water is a Salmonidae.

Preferably, said Salmonidae is selected from the group consisting of Atlantic salmon, Coho salmon, Chinook, rainbow trout, Arctic char and other farmed salmon species.

Preferably, the compound reduces the attraction between a parasite and said fish.

Preferably, said parasite is an ectoparasite, preferable a copepodid ectoparasite.

Preferably, said ectoparasite is sea lice *(Lepeophtheirus salmonis, Caligus* sp.*)*.

Preferably, R1 is a C₁-C₄ alkyl.

Preferably, said compound is butyl isothiocyanate.

Preferably, said compound is propyl isothiocyanate.

Preferably, R1 is a C₂-C₃ alkenyl.

Preferably, said compound is alkyl isothiocyanate.

Preferably, said phenyl alkyl is phenyl methyl, phenyl ethyl or phenyl propyl.

Preferably, said phenyl alkyl is phenyl ethyl.

Preferably, said compound is allyl-, propyl-, butyl- pentenyl-, phenyethyl-isothiocyanates.

Preferably, said compound is 2-phenyl ethyl isothiocyanate.

A second aspect of the present invention relates to a compound for use in reducing the attraction between a parasite and a fish, or for reducing the infestation or infection of a parasite in a fish, or for the treatment of a parasite infection in a fish, characterized in that said compound is added to said water or is administered to a fish in said water, wherein said compound is a compound of formula (II);

R¹-N=C=S (II)

wherein R¹ is C₁-C₄ alkyl or C₂-C₃ alkenyl or C₂-C₃-alkynyl or phenyl alkyl.

Preferably, said fish is a Salmonidae.

Preferably, said water is Salmonidae conditioned sea water or said fish in the water is a Salmonidae.

Preferably, said Salmonidae is selected from the group consisting of Atlantic salmon, Coho salmon, Chinook, rainbow trout, Arctic char and other farmed salmon species.

Preferably, said Salmonidae is Atlantic salmon.

Preferably, said Salmonidae is rainbow trout.

Preferably, the method reduces the attraction between a parasite and said fish.

Preferably, said parasite is an ectoparasite, preferable a copepodid ectoparasite.

Preferably, said ectoparasite is sea lice *(Lepeophtheirus salmonis, Caligus* sp.).

Preferably, R1 is a C₁-C₄ alkyl.

Preferably, said compound is butyl isothiocyanate.

Preferably, said compound is propyl isothiocyanate.

Preferably, R1 is a C₂-C₃ alkenyl.

Preferably, said compound is alkyl isothiocyanate.

Preferably, said phenyl alkyl is phenyl methyl, phenyl ethyl or phenyl propyl.

Preferably, said phenyl alkyl is phenyl ethyl.

Preferably, said compound is allyl-, propyl-, butyl-, pentenyl-, phenyethyl-isothiocyanates

Preferably, said compound is 2-phenyl ethyl isothiocyanate.

A third aspect of the present invention relates to a feed composition comprising conventional feed ingredients such as lipids, proteins, vitamins, carbohydrates and minerals, characterized in that the feed comprises a compound of formula (II);

**R¹-N=C=S** (II)

wherein R¹ is C₁-C₄ alkyl or C₂-C₃ alkenyl or C₂-C₃-alkynyl or phenyl alkyl.

Preferably, R1 is a C₁-C₄ alkyl.

Preferably, said compound is butyl isothiocyanate.

Preferably, said compound is propyl isothiocyanate.

Preferably, R1 is a C₂-C₃ alkenyl.

Preferably, said compound is alkyl isothiocyanate.

Preferably, said phenyl alkyl is phenyl methyl, phenyl ethyl or phenyl propyl.

Preferably, said phenyl alkyl is phenyl ethyl.

Preferably, said compound is 2-phenyl ethyl isothiocyanate.

Preferably, said compound is allyl-, propyl-, butyl-, pentenyl-, phenylethyl-isothiocyanates.

Preferably, said compound or extract in the feed are in a concentration range of 0.01-0,5, preferably in a concentration of 0.125% by weight of the feed.

A fourth aspect of the present invention relates to the use of a compound for the preparation of a pharmaceutical composition for the prevention and/or treatment of a parasite infection in fish, preferable a Salmonidae, wherein said compound is a compound of formula (II);

**R¹-N=C=S** (II)

wherein R¹ is C₁-C₄ alkyl or C₂-C₃ alkenyl or C₂-C₃-alkynyl or phenyl alkyl.

Preferably, the compound or extract is used for the manufacturing of a pharmaceutical or nutraceutical composition, or functional food.

### DESCRIPTION OF THE INVENTION

Embodiments of the invention will now be described, by the way of examples with reference to the following figures:
Figure 1 a shows the directional dose response of *L. salmonis* copepodids to seawater control, salmon conditioned water (SCW) and SCW plus 4-pentenyl, 2-phenylethyl and butyl isothiocyanate at 100 ppt.
Figure 1b shows the activation dose response of *L. salmonis* copepodids to seawater control, salmon conditioned water (SCW) and SCW plus 4-pentenyl, 2-phenylethyl and butyl isothiocyanate at 100 ppt.
Figure 2a shows the directional dose response of *L. salmonis* copepodids to seawater control, salmon conditioned water (SCW) and SCW plus 100 ppt propyl isothiocyanate.
Figure 2b shows the activation dose response of *L. salmonis* copepodids to seawater control, salmon conditioned water (SCW) and SCW plus 100 ppt propyl isothiocyanate.
Figure 3a shows the directional dose response of *L. salmonis* copepodids to seawater control, salmon conditioned water (SCW) and SCW plus butyl isothiocyanate at 1, 10 and 100 parts per trillion (ppt).
Figure 3b shows the activation dose response of *L. salmonis* copepodids to seawater control, salmon conditioned water (SCW) and SCW plus butyl isothiocyanate at 1, 10 and 100 parts per trillion (ppt).
Figure 4a shows the directional dose response of *L. salmonis* copepodids to seawater control, salmon conditioned water (SCW) and SCW plus allyl isothiocyanate at 1, 10 and 100 parts per trillion (ppt).
Figure 4b shows the activation dose response of *L. salmonis* copepodids to seawater control, salmon conditioned water (SCW) and SCW plus allyl isothiocyanate at 1, 10 and 100 parts per trillion (ppt).
Figure 4c shows the directional dose response of *L. salmonis* copepodids to seawater control, salmon conditioned water (SCW) and SCW plus allyl isothiocyanate at 1, 10 and 100 parts per trillion (ppt).
Figure 4d shows the directional dose response of *L. salmonis* copepodids to seawater control, salmon conditioned water (SCW) and SCW plus allyl isothiocyanate at 1, 10 and 100 parts per trillion (ppt).
Figure 4e shows the aactivation dose response of *L. salmonis* copepodids to seawater control, salmon conditioned water (SCW) and SCW plus allyl isothiocyanate at 1, 10 and 100 parts per trillion (ppt), blocks 1 and 2 combined.
Figure 5. Chemotaxis response of *C*. *rogercresseyi* Copepodid to stimulus masked with compounds B1(A), B2(B) y B3(C) at different concentration (* P < 0,05; Chi-square test)
Figure 6. Preference Index of *C*. *rogercresseyi* copepodids to host signal masked with compounds B1(A), B2 (B) y B3(C) at different concentration.
Figure 7. Fish fed the butyl isothiocyanate (B1) showed a significant reduction of 42% in levels of sea lice compared to controls (Figure 2). There was a trend for a reduction in lice levels with both diallyl sulfide (B2) and diallyl disulfide (B3)

### EXPERIMENTAL SECTION

### Example 1: In vitro assessment of the effect of different compounds on Lepeophtheirus salmonis

A number of plant products were tested for their ability to mask salmon odour in order to inhibit the attractant of lice to salmon and to prevent *L.salmonis* settlement on salmon. A Y-tube behavioural arena was developed and used to test the ability of plant extracts/compounds to inhibit copepodid attraction to salmon conditioned water.

Products tested were:
- garlic constituents; garlic oil, diallyl disulphide and diallyl sulphide
- cruciferous isothiocyanates; allyl-, propyl-, butyl-, pentenyl-, phenyethyl-isothiocyanates
- plant extracts; bog myrtle, lavender, rosemary

### Material and methods:

### Lice collection

Ovigerous female *Lepeophtheirus salmonis* were collected from Atlantic salmon. Material was transported on ice to the laboratory with clean seawater for sorting. Water from the source site was collected and used for subsequent rearing of egg strings. Strings were removed gently from their point of attachment to adult females using ultra-fine forceps and placed in 2 L glass conical flasks. All flasks were aerated to keep the strings in suspension and promote hatching. Egg strings were reared under a 16 h light - 8 h dark regime and at 12°C ambient temperature in water from the source site.

Development of the egg to the copepodid was determined as a function of the mean temperature following Johnson and Albright (1991). Strings were monitored twice daily for hatching of nauplii and subsequent development to the copepodid stage, at which point they were removed for use in behavioural bioassays.

### Fish Conditioned Water

Fish conditioned water was collected as described by Devine *et al.* (2000) and Ingvarsdottir *et al.* (2002b). Atlantic salmon, *S*. *salar* were maintained in aquaria containing artificial seawater (32 ‰). Fish conditioned water was obtained by placing the fish for 24 h into a circulating flume (20 cm x 25 cm x 420 cm) filled with artificial seawater (100 L) circulated at a rate of 30 cm s⁻¹. Aeration was provided by bubbling compressed air into the raceway. Standardisation of fish odour in the water was achieved by using the water at a concentration of 8-10 g live fish L⁻¹ 24 h⁻¹. Conditioned water was either used immediately or frozen for later use.

### Lice Behaviour L. salmonis

A vertical Y-tube bioassay modified by Bailey *et al.* (2006) from that previously described by Ingvarsdóttir *et al.* (2002a) was used to study *L. salmonis* copepodid activation and directional (taxis) responses to host semiochemical components and potential host-masking compounds. The Y-tube was constructed from glass (1 cm diameter bore) moulded into a 'Y' design between two glass sheets of glass (2 mm thick). The arms were 6.5 cm in length and the main leg was 8 cm long. The main leg of the Y-tube was fitted with a glass stopper and filter to prevent copepodids from entering the outflow tubing running to waste. A syringe pump (SP 200 iz, World Precision Instruments, Florida, USA) held two plastic 60 mL syringes (Terumo Monoject, New Jersey, USA), which were loaded with test odours prior to use. The syringe pump was programmed to deliver a consistent flow rate of 2 mL min⁻¹. Chemical dyes demonstrated a clear demarcation of the flow down each arm and no mixing of water in the main leg of the T-tube.

When single chemical stimuli were tested e.g. salmon conditioned water (SCW), the test water was introduced to one arm whilst artificial seawater (ASW) at 32 ‰ was introduced into the other. When one of the isothiocyanates for example was tested, seawater was introduced into one arm whilst SCW plus the isothiocyanate at the desired concentration were introduced to the other. The introduction of stimuli was alternated between left and right inflow arms during each experiment, with washing in between, to eliminate positional bias. At the beginning of each experiment, the Y-tube was allowed to fill and run with seawater or seawater plus a cue/masking chemical, and a single copepodid was introduced using PTFE tubing and syringe into the tube at a point 1.5 cm above the base of the main leg. The copepodid was allowed a maximum of 3 min to respond. Each trial consisted of 1 copepodid.

Replicate tests were carried out over a period of four days to monitor for age effects of the lice on results.

Behaviour was defined by the degree of movement within the Y-Tube, as described by Ingvarsdóttir *et al.* (2002b). Behaviour was divided into two categories, low and high. Low activity was defined as the movement of the copepodid less than the length of the main leg. High activity was defined as movement of the copepodid more than the length of the main leg. Movement into either arm was also regarded as high activity. Both activation and directional responses of copepodids were measured. For directional responses, the number of copepodids choosing the stimulus arm rather than the control arm within the allocated 3 min period were compared to the control in which seawater was presented in both arms.

### Chemicals

Chemicals used in behavioural bioassays were supplied by the Chemical Ecology Group at Rothamsted Research, Harpenden, Hertfordshire, UK. Solutions of individual chemicals in ethanol (0.001, 0.01, 0.1 and 1 mg/mL) were prepared and diluted to 1 µL/L in artificial seawater (Ingvarsdóttir *et al.,* 2002b) to give a final concentration of 0.1, 1, 10, 100 and 1,000 parts per trillion (ppt) respectively.

### Data Analysis

Copepodid responses to ASW (Artificial sea water) and SCW (Salmon conditioned sea water) across all experiment days were compared in the first instance using a chi-square test to determine if there was a day effect on louse behaviour. If this proved to be non-significant, it implies that the data are consistent across days and therefore can be pooled.

For directional responses and experiments on activity, the null hypothesis that all lice in all treatments behaved the same was tested using a 'global'·χ² contingency table (Zar, 1999). Upon rejection of that hypothesis, data were analysed by *post hoc* targeted pairwise comparisons using a 2 x 2 χ² contingency table (Zar, 1999) to identify whether pairs of treatments of interest were significantly different.

Experiments testing whether allyl isothiocyanate can mask the attractiveness of salmon conditioned water were conducted in two blocks. In addition to χ² analysis of the original data (block 1), binomial logistic regression was used to test whether copepodid directional and activation responses differed both between experimental treatments (salmon conditioned water presented alone, or with three concentrations of ally isothiocyanate, against an artificial seawater control) and between blocks. Two separate models were constructed, with either copepodid directional response (test or control) or activity (high or low) entered as the dependent variable. In both cases, treatment and block were entered as factors, with a treatment by block interaction included to test if louse responses to each treatment varied between blocks. Significance of terms in both models was investigated through stepwise deletion (changes in deviance assessed through χ² tests) and comparisons of responses at each concentration of allyl isothiocyanate with respect to salmon conditioned water (no allyl isothiocyanate) made using Wald statistics.

### Results in vitro assessment Lepeophtheirus salmonis

### Isothiocyanate Compounds

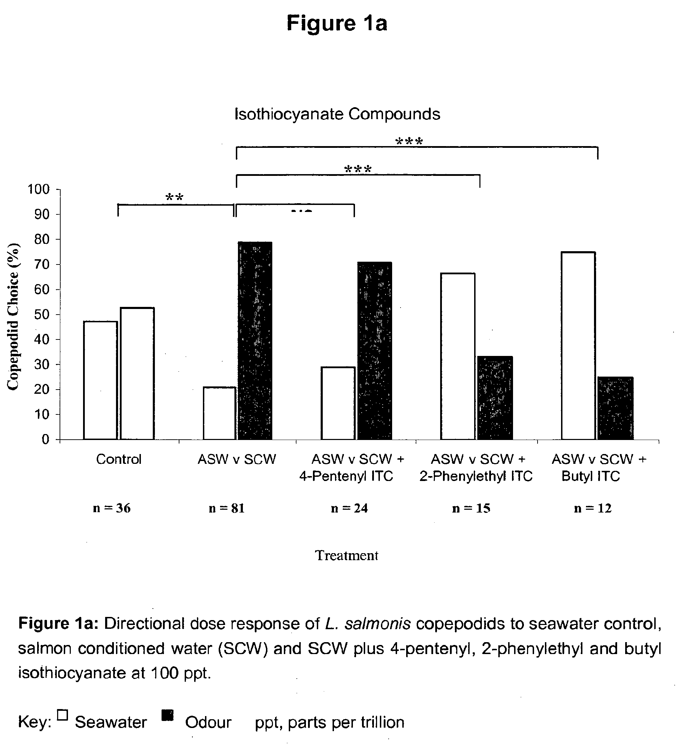

The global χ² showed that lice did not behave the same in all treatments (χ² = 26.50, df = 4, P < 0.001) in directional response assays. When compared with the seawater control, significantly more copepodids chose the arm containing the salmon conditioned water, SCW (χ² = 11.82, df = 1, P < 0.001). A significant decrease in copepodid responses was detected with SCW plus 100 ppt 2-phenylethyl (χ² = 13.06, df = 1, P < 0.001) and SCW plus 100 ppt butyl isothiocyanate (χ² = 15.14, df = 1, P < 0.001) when compared against SCW responses. No difference in directional responses was detected between SCW plus 100 ppt 4-pentenyl isothiocyanate and SCW responses (χ² = 0.7, df = 1, NS; Figure 1a). The number of *L. salmonis* copepodids making directional responses, not choosing and the total number of replicates for each treatment are presented in Table 2d.

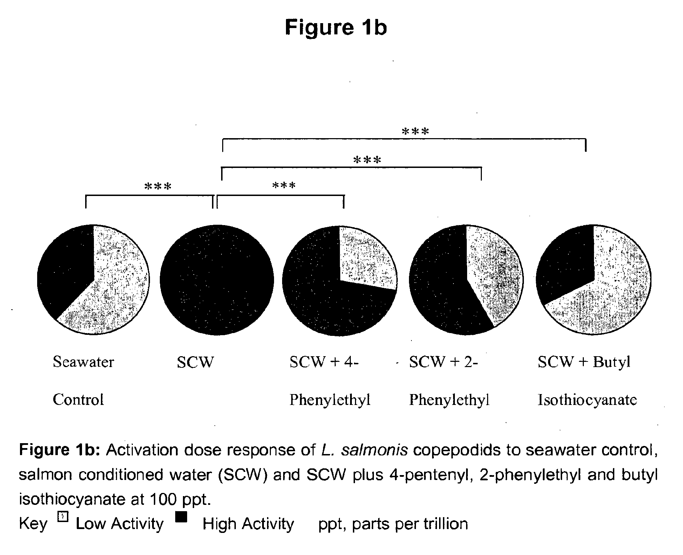

The global χ² showed that lice did not behave the same in all activity treatments (χ² = 97.56, df = 4, P < 0.001). When compared with the seawater control, a significant increase in high activity was detected with SCW (χ² = 80.54, df = 1, P < 0.001). Significantly more copepodids showed low activity with SCW plus 100 ppt 4-pentenyl (χ² = 25.97, df = 1, P < 0.001), 100 ppt 2-phenylethyl (χ² = 41.40, df = 1, P < 0.001) and 100 ppt butyl isothiocyanate (χ² = 75.42, df = 1, P < 0.001) when compared against SCW responses (Figure 1 b).

**Table 2d**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 36 | 84 | 120 |
| ASW v SCW | 81 | 2 | 83 |
| ASW v SCW + 4-Pentenyl Isothiocyanate | 24 | 26 | 50 |
| ASW v SCW + 2-Phenylethyl Isothiocyanate | 15 | 35 | 50 |
| ASW v SCW + Butyl Isothiocyanate | 12 | 40 | 52 |

### Propyl Isothiocyanate

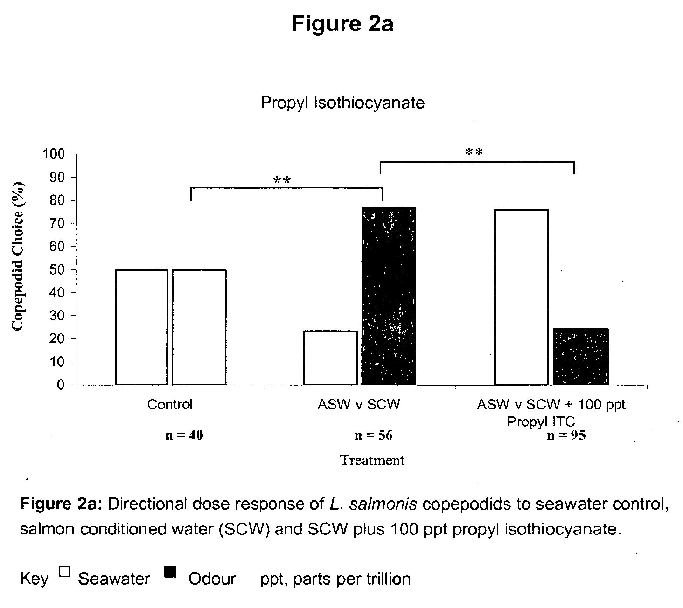

The global χ² showed that lice did not behave the same in all treatments (χ² = 39.84, df = 2, P < 0.001) in directional response assays. When compared with the seawater control, significantly more copepodids chose the arm containing the salmon conditioned water, SCW (χ² = 7.42, df = 1, P < 0.01). A significant decrease in copepodid responses was detected with SCW plus 100 ppt propyl isothiocyanate (χ² = 39.58, df = 1, P < 0.001) when compared against SCW responses (Figure 2a). The number of *L*. *salmonis* copepodids making directional responses, not choosing and the total number of replicates for each treatment are presented in Table 2e.

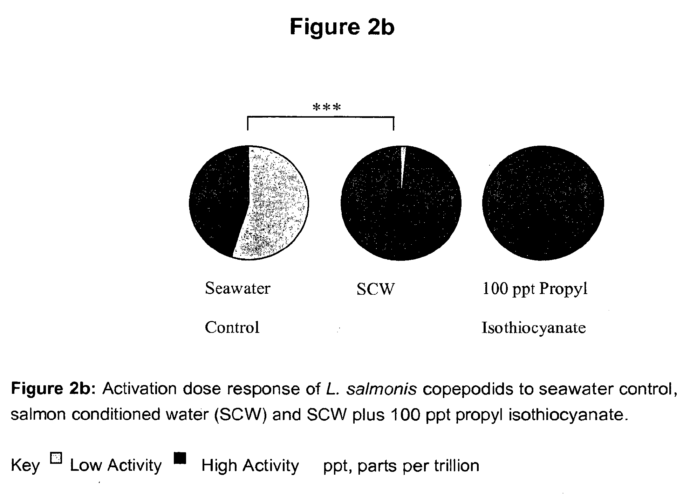

The global χ² showed that lice did not behave the same in all activity treatments (χ² = 59.78, df = 2, P < 0.001). When compared with the seawater control, a significant increase in high activity was detected with SCW (χ² = 26.69, df = 1, P < 0.001). No difference in activity was detected between SCW plus 100 ppt propyl isothiocyanate however (χ² = 0, df = 1, NS; Figure 2b).

**Table 2e**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 40 | 113 | 153 |
| ASW v SCW | 56 | 0 | 56 |
| ASW v SCW + 100 ppt Propyl Isothiocyanate | 95 | 5 | 100 |

### Butyl Isothiocyanate Dose Response

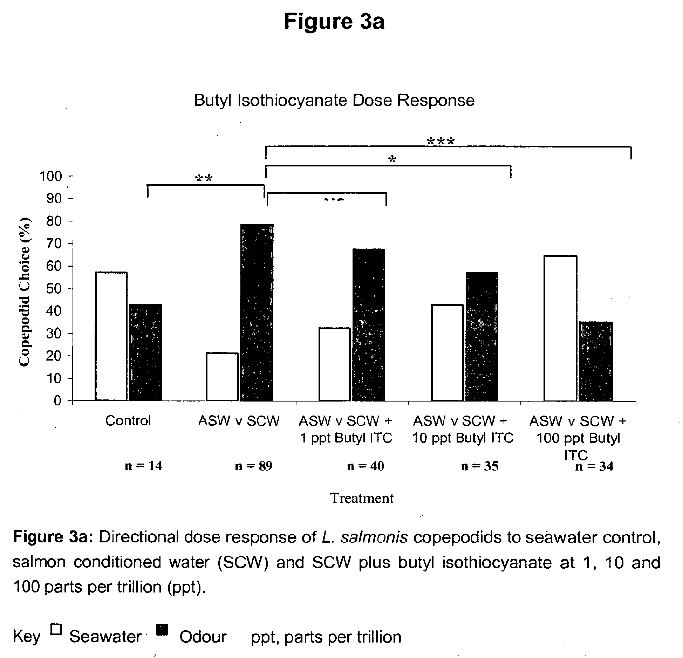

The global χ² showed that lice did not behave the same in all treatments (χ² = 23.99, df = 4, P < 0.001) in directional response assays. When compared with the seawater control, significantly more copepodids chose the arm containing the salmon conditioned water, SCW (χ² = 8.01, df = 1, P < 0.01). A significant decrease in copepodid responses was detected with SCW plus 10 ppt (χ² = 5.84, df = 1, P < 0.05) and 100 ppt butyl isothiocyanate (χ² = 20.81, df = 1, P < 0.001) when compared against SCW responses. However, no difference in directional responses was detected between SCW plus 1 ppt butyl isothiocyanate and SCW responses (χ² = 1.84, df = 1, NS; Figure 3a). The number of *L. salmonis* copepodids making directional responses, not choosing and the total number of replicates for each treatment are presented in Table 2f.

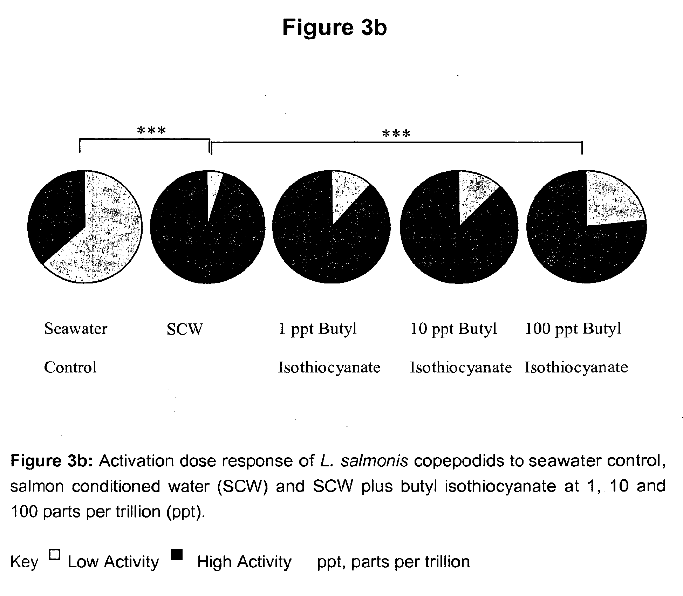

The global χ² showed that lice did not behave the same in all activity treatments (χ² = 91.94, df = 4, P < 0.001). When compared with the seawater control, a significant increase in high activity was detected with SCW (χ² = 75.04, df = 1, P < 0.001). Significantly more copepodids showed low activity with SCW plus 100 ppt butyl isothiocyanate (χ² = 15.43, df = 1, P < 0.001) when compared against SCW responses. No difference in activity was detected between SCW plus 1 and 10 ppt butyl isothiocyanate however (χ² = 2.65 and 2.64 respectively, df = 1, NS; Figure 3b).

**Table 2f**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 14 | 51 | 65 |
| ASW v SCW | 89 | 31 | 120 |
| ASW v SCW + 1 ppt Butyl Isothiocyanate | 40 | 20 | 60 |
| ASW v SCW + 10 ppt Butyl Isothiocyanate | 35 | 25 | 60 |
| ASW v SCW + 100 ppt Butyl Isothiocyanate | 34 | 31 | 65 |

### Allyl Isothiocyanate Dose Response

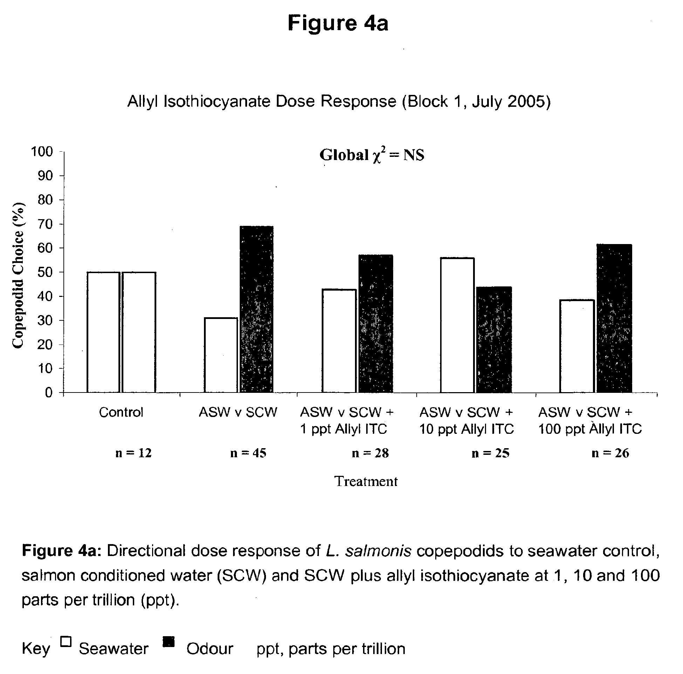

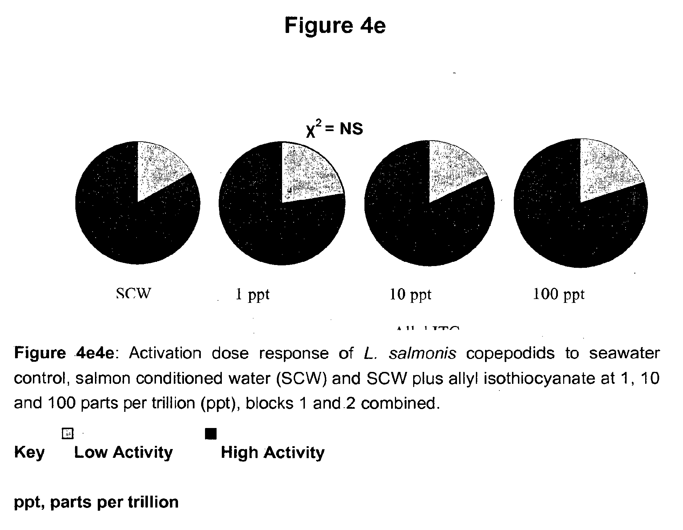

The global χ² showed that lice behaved the same in all treatments (χ² = 4.65, df = 4, NS) in directional response assays. As a result, further pairwise comparisons were not carried out (Figure 4a). The number of *L. salmonis* copepodids making directional responses, not choosing and the total number of replicates for each treatment are presented in Table 2g.

**Table 2g**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 12 | 33 | 45 |
| ASW v SCW | 45 | 16 | 61 |
| ASW v SCW + 1 ppt Allyl Isothiocyanate | 28 | 22 | 50 |
| ASW v SCW + 10 ppt Allyl Isothiocyanate | 25 | 25 | 50 |
| ASW v SCW + 100 ppt Allyl Isothiocyanate | 26 | 24 | 50 |

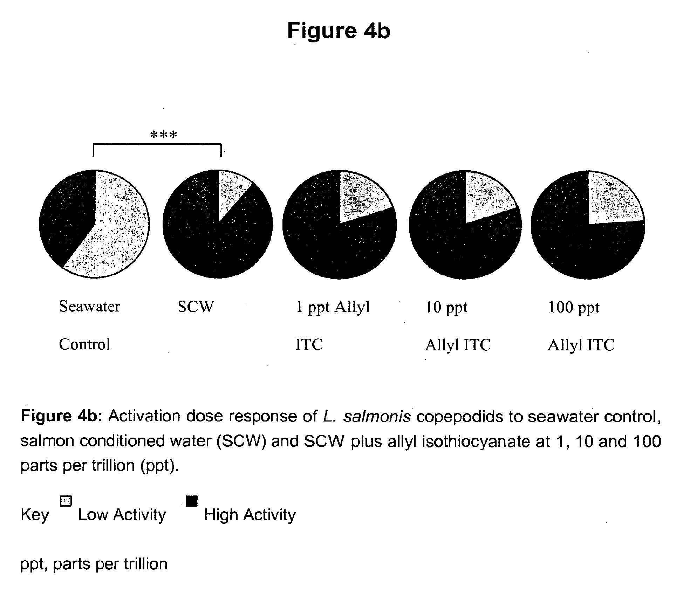

The global χ² showed that lice did not behave the same in all activity treatments (χ² = 37.24, df = 4, P < 0.001). When compared with the seawater control, a significant increase in high activity was detected with SCW (χ² = 27.99, df = 1, P < 0.001). No difference in activity was detected between SCW plus 1, 10 or 100 ppt allyl isothiocyanate and SCW responses however (χ² = 2.24, 1.54, 3.04 respectively, df = 1, NS; Figure 4b).

### Allyl Isothiocyanate Dose Response - Updated Analysis

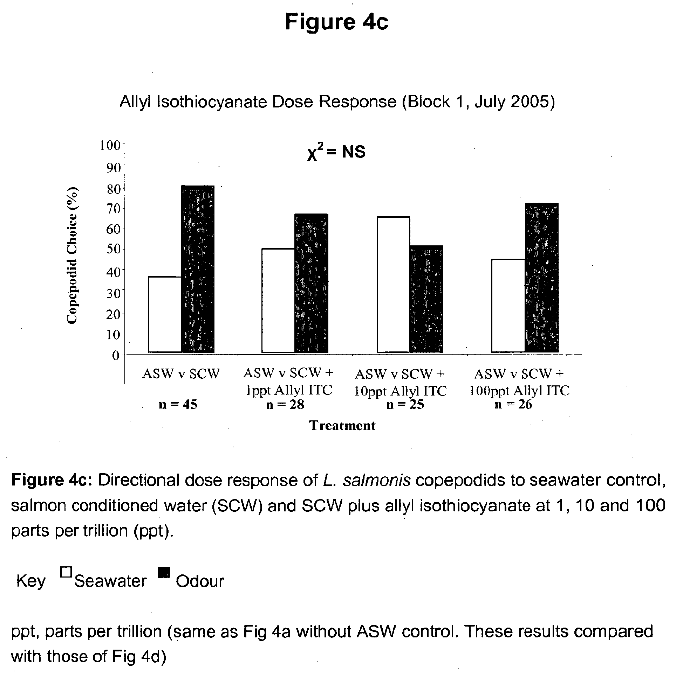

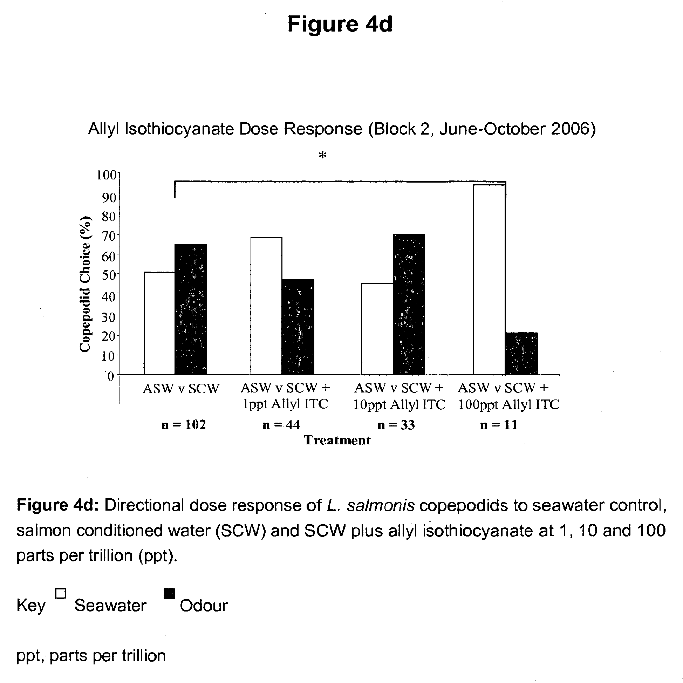

The effect of treatment on louse directional responses was found to differ between blocks, as demonstrated by a significant treatment by block interaction term (χ² = 8.24, df = 3, P < 0.05). While no overall difference in louse behaviour was found between treatments in experiments conducted in block 1 (July 2005; χ² = 4.24, df = 3, NS; Figure 4c), an overall effect of treatment was found in block 2 (June-October 2006; χ² = 9.11, df = 3, P < 0.05; Figure 4d), with fewer copepodids chose the test arm in the presence of 100 ppt allyl isothiocyanate than SCW presented unmasked (Wald = 4.65, df = 1, P < 0.05; Figure 4c).

There was no significant effect of block by treatment (χ² = 5.09, df =3, NS) or block (χ² = 0.001, df = 1, NS) on activation responses, allowing data to be pooled across blocks. No subsequent overall difference in activation responses was found across treatments (χ² = 1.90, df =3, NS; Figure 4d). The number of *L. salmonis* copepodids making directional responses, not choosing and the total number of replicates for each treatment are presented in Table 2h.

**Table 2h**

| Number of *L. salmonis* copepodids making directional responses, non-choosers and the total number of replicates for each treatment (blocks 1 and 2). | | | |
|---|---|---|---|
| Assay | Directional Responses | No Choice | Total No. Replicates |
| ASW v SCW | 147 | 68 | 215 |
| ASW v SCW + 1 ppt Allyl Isothiocyanate | 72 | 66 | 138 |
| ASW v SCW + 10 ppt Allyl Isothiocyanate | 58 | 44 | 102 |
| ASW v SCW + 100 ppt Allyl Isothiocyanate | 37 | 29 | 66 |

### Discussion:

In this study, it has been shown that copepodid larvae of the salmon louse, *L*. *salmonis,* show significant directional responses to isophorone, a component of salmon conditioned water. Isophorone has been identified as a behaviourally active component of salmon-conditioned water (Bailey *et al.,* 2006) and was therefore used as a host cue to elicit a response in preliminary experiments.

We have shown that 2-phenylethyl, butyl and propyl isothiocyanate at the 100 parts per trillion concentration, removed the attraction of copepodids to salmon conditioned water. 4-pentenyl isothiocyanate did not mask copepodid responses to salmon conditioned water however. Dose response experiments with butyl isothiocyanate showed 100 parts per trillion to be the most effective concentration for switching off responses to salmon conditioned water. Allyl isothiocyanate dose response assays suggest a possible effect at 100 parts per trillion.

A high number of non-choosers were seen in all seawater controls and is due to a lack of cues to stimulate a behavioural response from the lice.

In general, the seawater controls showed predominantly low activity behaviour in copepodids. This switched to high activity in the presence of a positive cue i.e. either isophorone or salmon conditioned water. Low activity re-appeared in the profile when test compounds were introduced, suggesting that the chemicals masked the effect of the isophorone or salmon conditioned water in copepodids. The extent of masking was variable between compounds and is thought to be related to the original field source of *L. salmonis.*

### Conclusions from example 1:

The use of plant derived masking compounds has been shown to significantly disrupt *L. salmonis* copepodid attraction to host (salmon) conditioned water *in vitro.* By masking the profile of the key host recognition molecules it was surprisingly possible to significantly reduce the host response of both *L. salmonis* and C. *rogercresseyi.* In the shown series of Y-tube assessments, sea lice showed a significant activity towards host odours from control Atlantic salmon. Inclusion of a series of masking compounds of vegetable origin effectively reduced this response in both species. Diallyl sulphide, diallyl disulphide, butyl isothiocyanate, allyl isothiocyanate, propyl Isothiocyanate, rosemary oil, lavender oil and bog myrtle were identified as candidate compounds for masking salmon host compounds.

The following compounds and concentrations were especially promising: Diallyl sulphide (10parts per trillion), diallyl disulphide (100parts per trillion), butyl isothiocyanate (100 parts per trillion), propyl isothiocyanate (100 parts per trillion) rosemary oil (100 parts per trillion), lavender oil (100 parts per trillion) and bog myrtle (1,000 parts per trillion).

### Example 2

### Evaluation of the effect of masking compounds on chemicals cues released by Atlantic salmon

### Material and methods

### Fish conditioned water

Atlantic salmon, *S*. *salar* were hatchery-reared stock produced at the west coast of Puerto Montt (Chile). For the preparation of Salmon Conditioned Water (SCW), one fish-host (100-200 g) was placed in a flume during 24 h with artificial seawater (100 L) (Aquarium salt; SERA, Heinsberg/Germany) with a salinity of 32‰ at 12 °C. The flow rate in the flume was 30 cm s-1 (Ingvarsdottir et al., 2002b). The water kept in the flume was used for bioassays, or frozen for use in chemical analysis.

### Lice

Ovigerous C. rogercresseyi females were collected from freshly harvested Atlantic salmon, on commercial fish farms on the west coast of Puerto Montt (Chile). Egg strings were removed gently from their point of attachment to adult females using ultra-fine forceps and were placed in a 500 mL glass culture flask with artificial seawater and held in suspension by an air supply through the stem at 12 °C keep them in absolute darkness until the copepodid stage was reached.

### Semiochemical Masking Compounds

Butyl isothiocyanate (B1), Diallyl sulphide (B2) and Diallyl disulfide (B3) were selected as test compounds. Each of the compounds were prepared in three solution with ethanol 1.0; 0.01 and 0.001 mg mL⁻¹, then where diluted to 1 µL L-1 in Salmon Conditioned Water (SCW).

### Preference Bioassays

A vertical Y-tube bioassay modified from that previously described was used to study *C. rogercresseyi* copepodid activation and directional (taxis) responses to host odours. The Y tube was made from perspex. The arms were 5 cm in length and the main leg was 6 cm long.

Water flowed through into each arm from reservoirs positioned immediately above the Y tube at a rate of 2 mL·min-1. In control assays, artificial seawater was introduced into both arms of the Y tube. When salmon-conditioned water (SCW) plus masking compounds were tested, the test water was introduced into one arm while seawater was introduced into the other.

At the beginning of each experiment, the Y tube was allowed to fill, and a single copepodid was introduced by polytetrafluoroethylene tubing (1 mm internal diameter (i.d.)) and syringe into the tube at a point 1 cm above the base of the main arm.

The copepodid was allowed a maximum of 5 min to respond. Behaviour was defined by the degree of movement within the Y tube, as described previously. Behaviour was divided into two categories, low and high. Low activity was defined as the movement of the copepodid less than the length of the main leg. High activity was defined as movement of the copepodid more than the length of the main leg. Preference was observed when the copepodid with high activity choose either arm. Both activation and directional responses of copepodids were measured. Each trial consisted of one copepodid, and each copepodid was never used more than once. There were 30-100 trials conducted for each experiment.

### Results

**Table 1**

| Response of copepodids Caligus rogercresseyi to different concentration of masking compounds in a vertical Y-tube bioassays. | | | | | |
|---|---|---|---|---|---|
| | Activity (%) | | | | N |
| | High | Low | X² | P | |
| Control | 67.50 | 32.50 | 4.9 | 0.027 | 40 |
| SCW | 62.64 | 37.36 | 5.8 | 0.016 | 100 |
| B1. Butyl isotiocianate | | | | | |
| SCW + (0,001 mg/mL) | 68.63 | 31.37 | 7.1 | 0.008 | 50 |
| SCW + (0,01 mg/mL) | 62.86 | 37.14 | 4.2 | 0.031 | 70 |
| SCW + (1,00 mg/mL) | 69.00 | 31.00 | 4.7 | 0.029 | 70 |
| B2. Dialyl sulfide | | | | | |
| SCW + (0,001 mg/mL) | 86.67 | 13.33 | 24.2 | 0.000 | 45 |
| SCW + (0,01 mg/mL) | 85.00 | 15.00 | 19.6 | 0.000 | 40 |
| SCW + (1,00 mg/mL) | 93.33 | 6.67 | 22.5 | 0.000 | 30 |
| B3. Dialyl disulfide | | | | | |
| SCW + (0,001 mg/mL) | 80.65 | 19.35 | 24.2 | 0.000 | 31 |
| SCW + (0,01 mg/mL) | 67.50 | 32.50 | 4.9 | 0.027 | 40 |
| SCW + (1,00 mg/mL) | 93.33 | 6.67 | 22.5 | 0.000 | 30 |
| P > 0,05 (test X²). | | | | | |

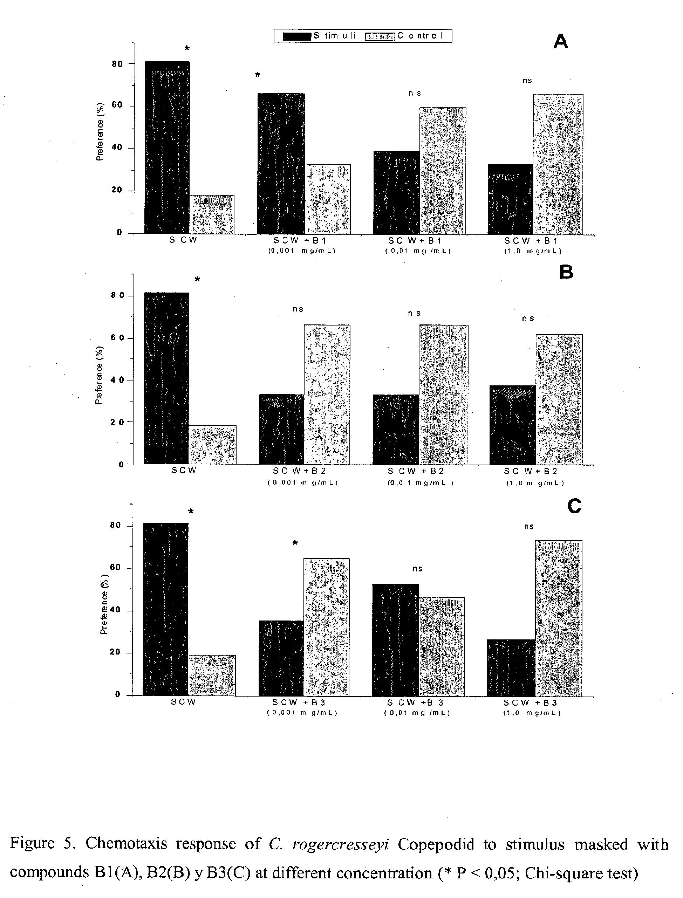

The level of preference was affected when the masking compounds were added. B1 at 0.01 and 1 mg / mL tends to change the preference shown by the sea lice at a lower concentration and control (Fig. 5A).

B2, at all the concentrations, showed a masking effect on the chemical cues released by Atlantic salmon, although no significant differences (Fig. 5B). B3 at 0.001 mg / mL significantly (P > 0.05) changed the preference of the copepodids (Fig. 5C).

An index of preference (IP) were calculated. IP = # visits at stimulus zones / # visits in the control zone. Which indicates, if IP = 1, there is no avoidance neither preference, if IP > 1, it indicates that there is preference for the stimulus, and if IP <1, it indicates an avoidance for the stimulus or a preference for control.

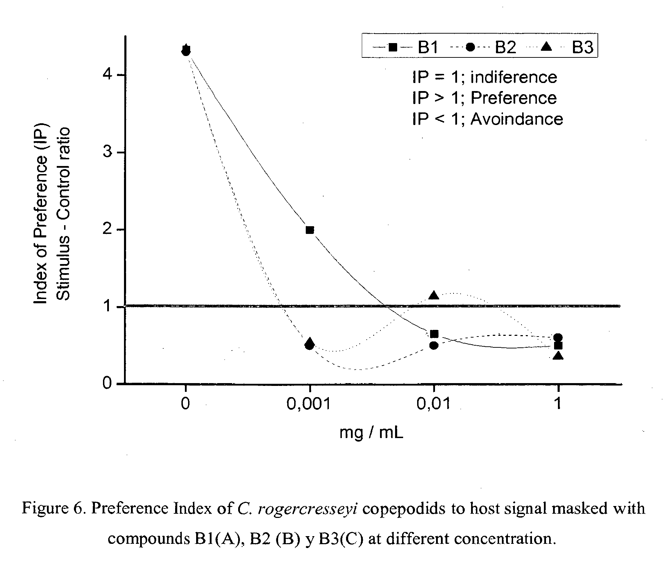

This study found that the IP calculated for B1 showed that the highest concentrations (0.01 and 1.0 mg mL-1) reduced the preference for the stimulus of Atlantic salmon. In the case of masked B2 and B3, the IP showed that both compounds were effective in their action of masking chemical cues (Fig. 6).

### Example 3

### Effects of B's in feed on disruption of copepodids settlement of Caligus rogercresseyi

The aim of this experiment was to validate the effect of three masking compounds in feeds on the disruption of copepodid settlement and *in vivo* challenge assays.

### Materials and Method

### Semiochemical Masking Compounds

Isobutyl thiocyanate (B1), Diallyl sulfide (B2) and Diallyl disulfide (B3) were selected as test compounds.

### Tank Trails

### Fish

Atlantic salmon, *Salmo salar* (N = 168; 500g avg), hatchery-reared stock produced and maintained in Chile prior to the experiment, were in Chile, smolted gradually through a freshwater to seawater gradient and held in a circular tank (12 m³). Fish were pit-tagged at the end of smoltification.

### Seal ice

100 ovigerous females *C*. *rogercresseyi* (5000 copepodids) for each tank were collected from freshly harvested Atlantic salmon, *S. salar* were placed in 2000 mL glass culture flasks with clean seawater, and held in suspension by an air supply through the stem at 12°C in absolute darkness. Egg strings were removed gently from their point of attachment using ultra-fine forceps and were placed in a 2000 mL glass culture flask with clean seawater and held in suspension with air supply at 12°C keep them in absolute darkness until the copepodid stage was reached. The emerged copepodid were used for infestation during the trail.

### Tank distribution.

14 fish, individually weight and tagged, were distributed in 12 fibreglass tanks (350L) with a flow through seawater (32 ‰) system at 13-14°C. Three tanks (replicas) were used for each masking compound dose and control diet.

### Masking compound feed formulation.

A dose of masking compounds (B1, B2 and B3) (0.125%) were tested against sea lice settlement compare with a commercial diet used as a Control. Feeding periods were held for 21 days, before sea lice infestation (Table 1). Post-Infestation feeding was held for 8 days.

**Table 1**

| Setting up experiments. | | | | |
|---|---|---|---|---|
| Feeding Tanks | Formulation | Number of Fish | Fish weight (g) | Feeding days) |
| 1, 2, 3 | FormB1 (0.125%) | 14 | 500 | 21 |
| 4, 5, 6 | FormB2 (0.125%) | 14 | 500 | 21 |
| 7, 8, 9 | FormB3 (0.125%) | 14 | 500 | 21 |
| 10, 11, 12 | Control | 14 | 500 | 21 |

### Sea lice Counting

Fish were culled and removed for the sampling. Sea lice were counted individually on each fish at 8 days post challenge.

### Results

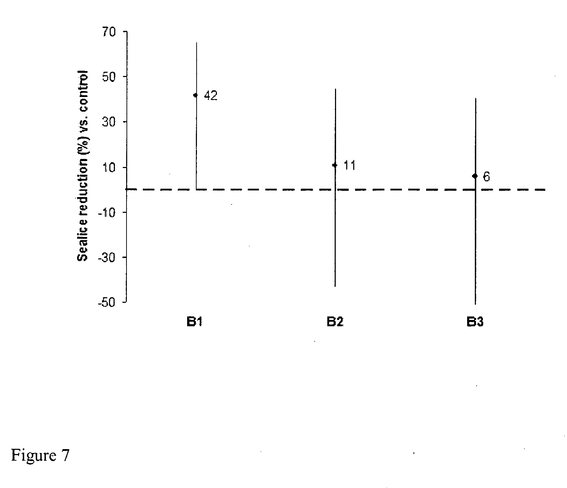

Fish fed the butyl isothiocyanate (B1) showed a significant reduction of 42% in levels of sea lice compared to controls (Figure 7). There was a trend for a reduction in lice levels with both diallyl sulfide (B2) and diallyl disulfide (B3)

It will be appreciated that the features of the invention described in the foregoing can be modified without departing from the scope of the invention.

### Definitions of terms:

The term "semiochemical" (semeon means a signal in Greek) is a generic term used for a chemical substance or mixture that carries a message. These chemicals acts as messengers for members of the same species or in some cases other species. It is usually used in the field of chemical ecology to encompass pheromones, allomones, kairomones, attractants and repellents. Please note especially that the term in respect of this application is not restricted to messengers between the same species, and that the term specifically is used to denote messengers between different species, such as between a Salmonidae and a parasite. The term is intended to include the chemical compounds which are specific for the attraction of parasites to Salmonidae, and especially to the attraction of sea lice to Salmonidae.

## Claims

1. A compound for use in masking the odor of a fish semiochemical in water in reducing the attraction between a parasite and a fish, **characterized in that** said compound is added to said water or is administered to a fish in said water, wherein said compound is a compound of formula (II);
R¹-N=C=S (II)
wherein R¹ is C₁-C₄ alkyl or C₂-C₃ alkenyl or C₂-C₃-alkynyl or phenyl alkyl.

2. Compound according to claim 1, wherein said fish semiochemical is isophorone.

3. Compound according to claim 1, wherein said fish semiochemical is 1-Octen-3-ol or 6-methyl-5-hepten-2-one.

4. Compound according to claim 1, wherein said fish is a Salmonidae.

5. Compound according to claim 1, wherein said water is Salmonidae conditioned sea water or said fish in the water is a Salmonidae.

6. Compound according to claim 4 or 5, wherein said Salmonidae is selected from the group consisting of Atlantic salmon, Coho salmon, Chinook, rainbow trout, Arctic char and other farmed salmon species.

7. Compound according to any of the claims 1-6, wherein the method reduces the attraction between a parasite and said fish.

8. Compound according to claim 7, wherein said parasite is an ectoparasite.

9. Compound according to claim 8, wherein said ectoparasite is sea lice *(Lepeophtheirus salmonis, Caligus* sp.*)*.

10. Compound according to claim 1, wherein R1 is a C₁-C₄ alkyl.

11. Compound according to claim 1, wherein said compound is butyl isothiocyanate.

12. Compound according to claim 1, wherein said compound is propyl isothiocyanate.

13. Compound according to claim 1, wherein R1 is a C₂-C₃ alkenyl.

14. Compound according to claim 1, wherein said compound is alkyl isothiocyanate.

15. Compound according to claim 1, wherein said phenyl alkyl is phenyl methyl, phenyl ethyl or phenyl propyl.

16. Compound according to claim 15, wherein said phenyl alkyl is phenyl ethyl.

17. Compound according to claim 1, wherein said compound is allyl-, propyl-, butyl-, pentenyl-, phenyethyl-isothiocyanates.

18. Compound according to claim 1, wherein said compound is 2-phenyl ethyl isothiocyanate.

19. A compound for use in reducing the attraction between a parasite and a fish, or for reducing the infestation or infection of a parasite in a fish, or for the treatment of a parasite infection in a fish, **characterized in that** said compound is added to said water or is administered to a fish in said water, wherein said compound is a compound of formula (II);
R¹-N=C=S (II)
wherein R¹ is C₁-C₄ alkyl or C₂-C₃ alkenyl or C₂-C₃-alkynyl or phenyl alkyl.

20. Compound according to claim 19, wherein said fish is a Salmonidae.

21. Compound according to claim 19, wherein said water is Salmonidae conditioned sea water or said fish in the water is a Salmonidae.

22. Compound according to claim 20, wherein said Salmonidae is selected from the group consisting of Atlantic salmon, coho salmon, Chinook, rainbow trout, Arctic charr and other farmed salmon species.

23. Compound according to claim 20, wherein said Salmonidae is Atlantic salmon.

24. Compound according to claim 20, wherein said Salmonidae is rainbow trout.

25. Compound according to any of the claims 19-24, wherein the method reduces the attraction between a parasite and said fish.

26. Compound according to claim 25, wherein said parasite is a copepodid ectoparasite.

27. Compound according to claim 26, wherein said ectoparasite is sea lice *(Lepeophtheirus salmonis, Caligus* sp.*)*.

28. Compound according to claim 19, wherein R1 is a C₁-C₄ alkyl.

29. Compound according to claim 19, wherein said compound is butyl isothiocyanate.

30. Compound according to claim 19, wherein said compound is propyl isothiocyanate.

31. Compound according to claim 19, wherein R1 is a C₂-C₃ alkenyl.

32. Compound according to claim 19, wherein said compound is alkyl isothiocyanate.

33. Compound according to claim 19, wherein said phenyl alkyl is phenyl methyl, phenyl ethyl or phenyl propyl.

34. Compound according to claim 33, wherein said phenyl alkyl is phenyl ethyl.

35. Compound according to claim 19, wherein said compound is allyl-, propyl-, butyl-, pentenyl-, phenyethyl-isothiocyanates

36. Compound according to claim 19, wherein said compound is 2-phenyl ethyl isothiocyanate.

37. A feed composition comprising conventional feed ingredients such as lipids, proteins, vitamins, carbohydrates and minerals, for use in reducing the attraction between a parasite and a fish **characterized in that** the feed comprises a compound of formula (II);
R¹-N=C=S (II)
wherein R¹ is C₁-C₄ alkyl or C₂-C₃ alkenyl or C₂-C₃-alkynyl or phenyl alkyl.

38. A feed composition according to claim 37, wherein R1 is a C₁-C₄ alkyl.

39. A feed composition according to claim 37, wherein said compound is butyl isothiocyanate.

40. A feed composition according to claim 37, wherein said compound is propyl isothiocyanate.

41. A feed composition according to claim 37, wherein R1 is a C₂-C₃ alkenyl.

42. A feed composition according to claim 37, wherein said compound is alkyl isothiocyanate.

43. A feed composition according to claim 37, wherein said phenyl alkyl is phenyl methyl, phenyl ethyl or phenyl propyl.

44. A feed composition according to claim 37, wherein said phenyl alkyl is phenyl ethyl.

45. A feed composition according to claim 44, wherein said compound is 2-phenyl ethyl isothiocyanate.

46. A feed composition according to claim 37, wherein said compound is allyl-, propyl-, butyl-, pentenyl-, phenylethyl-isothiocyanates.

47. A feed composition according to claim 37, said compound or extract in the feed are in a concentration range of 0.01-0,5, preferably in a concentration of 0.125% by weight of the feed.

48. Use of a compound for the preparation of a pharmaceutical composition for the prevention and/or treatment of a parasite infection in fish, preferably a Salmonidae, wherein said compound is a compound of formula (II);
R¹-N=C=S (II)
wherein R¹ is C₁-C₄ alkyl or C₂-C₃ alkenyl or C₂-C₃-alkynyl or phenyl alkyl.

## Patentansprüche

1. Verbindung zur Verwendung beim Überdecken des Geruchs einer Fisch-Semiochemikalie in Wasser zur Verringerung der Anziehung zwischen einem Parasiten und einem Fisch, **dadurch gekennzeichnet, dass** die Verbindung zum Wasser hinzugefügt oder einem Fisch in dem Wasser verabreicht wird, wobei die Verbindung eine Verbindung der Formel (II) ist:
R¹-N=C=S (II)
wobei R¹ C₁-C₄-Alkyl oder C₂-C₃-Alkenyl oder C₂-C₃-Alkynyl oder Phenylalkyl ist.

2. Verbindung nach Anspruch 1, wobei die Fisch-Semiochemikalie Isophoron ist.

3. Verbindung nach Anspruch 1, wobei die Fisch-Semiochemikalie 1-Octen-3-ol oder 6-Methyl-5-hepten-2-one ist.

4. Verbindung nach Anspruch 1, wobei der Fisch ein Salmonide ist.

5. Verbindung nach Anspruch 1, wobei das Wasser ein für Salmoniden aufbereitetes Meerwasser oder der Fisch in dem Wasser ein Salmonide ist.

6. Verbindung nach Anspruch 4 oder 5, wobei der Salmonide aus der Gruppe ausgewählt wird, die aus Atlantischem Lachs, Silberlachs, Königslachs, Regenbogenforelle, Seesaibling und anderen Zuchtlachsarten besteht.

7. Verbindung nach einem der Ansprüche 1-6, wobei das Verfahren die Anziehung zwischen einem Parasiten und dem Fisch verringert.

8. Verbindung nach Anspruch 7, wobei der Parasit ein Ektoparasit ist.

9. Verbindung nach Anspruch 8, wobei der Ektoparasit Lachslaus (*Lepeophtheirus salmonis, Caligus sp.*) ist.

10. Verbindung nach Anspruch 1, wobei R¹ ein C₁-C₄-Alkyl ist.

11. Verbindung nach Anspruch 1, wobei die Verbindung Butylisothiocyanat ist.

12. Verbindung nach Anspruch 1, wobei die Verbindung Propylisothiocyanat ist.

13. Verbindung nach Anspruch 1, wobei R¹ ein C₂-C₃-Alkenyl ist.

14. Verbindung nach Anspruch 1, wobei die Verbindung Alkylisothiocyanat ist.

15. Verbindung nach Anspruch 1, wobei das Phenylalkyl Phenylmethyl, Phenylethyl oder Phenylpropyl ist.

16. Verbindung nach Anspruch 15, wobei das Phenylalkyl Phenylethyl ist.

17. Verbindung nach Anspruch 1, wobei die Verbindung Allyl-, Propyl-, Butyl-, Pentenyl-, Phenyethylisothiocyanate ist.

18. Verbindung nach Anspruch 1, wobei die Verbindung 2-Phenylethylisothiocyanat ist.

19. Verbindung zur Verwendung bei der Verringerung der Anziehung zwischen einem Parasiten und einem Fisch, oder zur Verringerung des Befalls oder der Infektion mit einem Parasiten bei einem Fisch, oder zur Behandlung einer Parasiteninfektion bei einem Fisch, **dadurch gekennzeichnet, dass** die Verbindung zum Wasser hinzugefügt oder einem Fisch in dem Wasser verabreicht wird, wobei die Verbindung eine Verbindung der Formel (II) ist:
R¹-N=C=S (II)
wobei R¹ C₁-C₄-Alkyl oder C₂-C₃-Alkenyl oder C₂-C₃-Alkynyl oder Phenylalkyl ist.

20. Verbindung nach Anspruch 19, wobei der Fisch ein Salmonide ist.

21. Verbindung nach Anspruch 19, wobei das Wasser ein für Salmoniden aufbereitetes Meerwasser oder der Fisch in dem Wasser ein Salmonide ist.

22. Verbindung nach Anspruch 20, wobei der Salmonide aus der Gruppe ausgewählt wird, die aus Atlantischem Lachs, Silberlachs, Königslachs, Regenbogenforelle, Seesaibling und anderen Zuchtlachsarten besteht.

23. Verbindung nach Anspruch 20, wobei der Salmonide ein Atlantischer Lachs ist.

24. Verbindung nach Anspruch 20, wobei der Salmonide eine Regenbogenforelle ist.

25. Verbindung nach einem der Ansprüche 19-24, wobei das Verfahren die Anziehung zwischen einem Parasiten und dem Fisch verringert.

26. Verbindung nach Anspruch 25, wobei der Parasit ein Copepodid-Ektoparasit ist.

27. Verbindung nach Anspruch 26, wobei der Ektoparasit Lachslaus (*Lepeophtheirus salmonis, Caligus sp.*) ist.

28. Verbindung nach Anspruch 19, wobei R¹ ein C₁-C₄-Alkyl ist.

29. Verbindung nach Anspruch 19, wobei die Verbindung Butylisothiocyanat ist.

30. Verbindung nach Anspruch 19, wobei die Verbindung Propylisothiocyanat ist.

31. Verbindung nach Anspruch 19, wobei R¹ ein C₂-C₃-Alkenyl ist.

32. Verbindung nach Anspruch 19, wobei die Verbindung Alkylisothiocyanat ist.

33. Verbindung nach Anspruch 19, wobei das Phenylalkyl Phenylmethyl, Phenylethyl oder Phenylpropyl ist.

34. Verbindung nach Anspruch 33, wobei das Phenylalkyl Phenylethyl ist.

35. Verbindung nach Anspruch 19, wobei die Verbindung Allyl-, Propyl-, Butyl-, Pentenyl-, Phenyethylisothiocyanate ist.

36. Verbindung nach Anspruch 19, wobei die Verbindung 2-Phenylethylisothiocyanat ist.

37. Futterzusammensetzung, die übliche Futterbestandteile wie Lipide, Proteine, Vitamine, Kohlenhydrate und Mineralien enthält, zur Verwendung bei der Verringerung der Anziehung zwischen einem Parasiten und einem Fisch, **dadurch gekennzeichnet, dass** das Futter eine Verbindung der Formel (II) enthält:
R¹-N=C=S (II)
wobei R¹ C₁-C₄-Alkyl oder C₂-C₃-Alkenyl oder C₂-C₃-Alkynyl oder Phenylalkyl ist.

38. Futterzusammensetzung nach Anspruch 37, wobei R¹ ein C₁-C₄-Alkyl ist.

39. Futterzusammensetzung nach Anspruch 37, wobei die Verbindung Butylisothiocyanat ist.

40. Futterzusammensetzung nach Anspruch 37, wobei die Verbindung Propylisothiocyanat ist.

41. Futterzusammensetzung nach Anspruch 37, wobei R¹ ein C₂-C₃-Alkenyl ist.

42. Futterzusammensetzung nach Anspruch 37, wobei die Verbindung Alkylisothiocyanat ist.

43. Futterzusammensetzung nach Anspruch 37, wobei das Phenylalkyl Phenylmethyl, Phenylethyl oder Phenylpropyl ist.

44. Futterzusammensetzung nach Anspruch 37, wobei das Phenylalkyl Phenylethyl ist.

45. Futterzusammensetzung nach Anspruch 44, wobei die Verbindung 2-Phenylethylisothiocyanat ist.

46. Futterzusammensetzung nach Anspruch 37, wobei die Verbindung Allyl-, Propyl-, Butyl-, Pentenyl-, Phenylethylisothiocyanate ist.

47. Futterzusammensetzung nach Anspruch 37, wobei die Verbindung oder der Extrakt im Futter in einem Konzentrationsbereich von 0,01-0,5, vorzugsweise in einer Konzentration von 0,125 Gew.-% des Futters vorliegt.

48. Verwendung einer Verbindung für die Zubereitung einer pharmazeutischen Zusammensetzung zur Verhinderung und/oder Behandlung einer Parasiteninfektion in einem Fisch, vorzugsweise einem Salmoniden, wobei die Verbindung eine Verbindung der Formel (II) ist:
R¹-N=C=S (II)
wobei R¹ C₁-C₄-Alkyl oder C₂-C₃-Alkenyl oder C₂-C₃-Alkynyl oder Phenylalkyl ist.

## Revendications

1. Composé pour son utilisation pour masquer l'odeur d'une substance sémiochimique de poisson dans l'eau afin de réduire l'attraction entre un parasite et un poisson, **caractérisé en ce que** ledit composé est ajouté à ladite eau ou est administré à un poisson dans ladite eau, dans lequel ledit composé est un composé de formule (II) ;
R¹-N=C=S (II)
où R¹ est un alkyle en C₁-C₄ ou un alkényle en C₂-C₃ ou un alkynyle en C₂-C₃ ou un phénylalkyle.

2. Composé selon la revendication 1, dans lequel ladite substance sémiochimique de poisson est une isophorone.

3. Composé selon la revendication 1, dans lequel ladite substance sémiochimique de poisson est une 1-Octèn-3-ol ou 6-méthyl-5-heptèn-2-one.

4. Composé selon la revendication 1, dans lequel ledit poisson est un salmonidé.

5. Composé selon la revendication 1, dans lequel ladite eau est de l'eau de mer conditionnée pour les salmonidés ou lesdits poissons dans l'eau sont des salmonidés.

6. Composé selon la revendication 4 ou 5, dans lequel ledit salmonidés est choisi dans le groupe composé du saumon de l'Atlantique, du saumon coho, du saumon quinnat, de la truite arc-en-ciel, de l'omble chevalier et d'autres espèces de saumon d'élevage.

7. Composé selon l'une quelconque des revendications 1 à 6, dans lequel le procédé réduit l'attraction entre un parasite et ledit poisson.

8. Composé selon la revendication 7, dans lequel ledit parasite est un ectoparasite.

9. Composé selon la revendication 8, dans lequel ledit ectoparasite est un pou du poisson *(Lepeophtheirus salmonis, Caligus* sp).

10. Composé selon la revendication 1, dans lequel R1 est un alkyle en C₁-C₄.

11. Composé selon la revendication 1, dans lequel ledit composé est un isothiocyanate de butyle.

12. Composé selon la revendication 1, dans lequel ledit composé est un isothiocyanate de propyle.

13. Composé selon la revendication 1, dans lequel R1 est un alkényle **en C₂-C₃.**

14. Composé selon la revendication 1, dans lequel ledit composé est un isothiocyanate d'alkyle.

15. Composé selon la revendication 1, dans lequel ledit phénylalkyle est un phénylméthyle, un phényléthyle ou un phénylpropyle.

16. Composé selon la revendication 15, dans lequel ledit phénylalkyle est un phényléthyle.

17. Composé selon la revendication 1, dans lequel ledit composé est un isothiocyanate d'allyle, de propyle, de butyle, de pentényle, de phényéthyle.

18. Composé selon la revendication 1, dans lequel ledit composé est un isothiocyanate de 2-phényléthyle.

19. Composé pour son utilisation pour réduire l'attraction entre un parasite et un poisson ou pour réduire l'infestation ou l'infection par un parasite chez un poisson, ou dans le traitement de l'infection par un parasite chez un poisson, **caractérisé en ce que** ledit composé est ajouté à ladite eau ou est administré à un poisson dans ladite eau, dans lequel ledit composé est un composé de formule (II) ;
R¹-N=C=S (II)
où R¹ est un alkyle en C₁-C₄ ou un alkényle en C₂-C₃ ou un alkynyle en C₂-C₃ ou un phénylalkyle.

20. Composé selon la revendication 19, dans lequel ledit poisson est un salmonidé.

21. Composé selon la revendication 19, dans lequel ladite eau est une eau de mer conditionnée pour les salmonidés ou ledit poisson dans l'eau est un salmonidé

22. Composé selon la revendication 20, dans lequel ledit salmonidé est choisi dans le groupe composé du saumon de l'Atlantique, du saumon coho, du saumon quinnat, de la truite arc-en-ciel, de l'omble chevalier et d'autres espèces de saumons d'élevage.

23. Composé selon la revendication 20, dans lequel ledit salmonidé est un saumon de l'Atlantique.

24. Composé selon la revendication 20, dans lequel ledit salmonidé est une truite arc-en-ciel.

25. Composé selon l'une quelconque des revendications 19 à 24, dans lequel le procédé réduit l'attraction entre un parasite et ledit poisson.

26. Composé selon la revendication 25, dans lequel ledit parasite est un copépode ectoparasite.

27. Composé selon la revendication 26, dans lequel ledit ectoparasite est un pou du poisson *(Lepeophtheirus salmonis, Caligus* sp.).

28. Composé selon la revendication 19, dans lequel R1 est un alkyle en C₁-C₄.

29. Composé selon la revendication 19, dans lequel ledit composé est un isothiocyanate de butyle.

30. Composé selon la revendication 19, dans lequel ledit composé est un isothiocyanate de propyle.

31. Composé selon la revendication 19, dans lequel R1 est un alkényle en C₂-C₃.

32. Composé selon la revendication 19, dans lequel ledit composé est un isothiocyanate d'alkyle.

33. Composé selon la revendication 19, dans lequel ledit phénylalkyle est phénylméthyle, un phényléthyle ou un phénylpropyle.

34. Composé selon la revendication 33, dans lequel ledit phénylalkyle est un phényléthyle.

35. Composé selon la revendication 19, dans lequel ledit composé est un isothiocyanate d'allyle, de propyle, de butyle, de pentényle, de phényléthyle.

36. Composé selon la revendication 19, dans lequel ledit composé est un isothiocyanate de 2-phényléthyle.

37. Composition alimentaire comprenant des ingrédients alimentaires conventionnels tels que des lipides, des protéines, des vitamines, des glucides et des minéraux, pour son utilisation pour réduire l'attraction entre un parasite et un poisson, **caractérisée en ce que** l'alimentation comprend un composé de formule (II) ;
R¹-N=C=S (II)
où R¹ est un alkyle en C1-C4 ou un alkényle en C₂-C₃ ou un alkynyle en C₂-C₃ ou un phénylalkyle.

38. Composition alimentaire selon la revendication 37, dans laquelle R1 est un alkyle en C₁-C₄.

39. Composition alimentaire selon la revendication 37, dans laquelle ledit composé est un isothiocyanate de butyle.

40. Composition alimentaire selon la revendication 37, dans laquelle ledit composé est un isothiocyanate de propyle.

41. Composition alimentaire selon la revendication 37, dans laquelle R1 est un alkényle en C₂-C₃.

42. Composition alimentaire selon la revendication 37, dans laquelle ledit composé est un isothiocyanate d'alkyle.

43. Composition alimentaire selon la revendication 37, dans laquelle ledit phénylalkyle est un phénylméthyle, un phényléthyle ou un phénylpropyle.

44. Composition alimentaire selon la revendication 37, dans laquelle ledit phénylalkyle est un phényléthyle.

45. Composition alimentaire selon la revendication 44, dans laquelle ledit composé est un isothiocyanate de 2-phényléthyle.

46. Composition alimentaire selon la revendication 37, dans laquelle ledit composé est un isothiocyanate d'allyle, de propyle, de butyle, de pentényle, de phényléthyle.

47. Composition alimentaire selon la revendication 37, ledit composé ou extrait dans l'alimentation sont présents à une concentration de l'ordre de 0,01 à 0,5, de préférence à une concentration de 0,125 % en poids de l'aliment.

48. Utilisation du composé pour la préparation d'une composition pharmaceutique pour la prévention et/ou le traitement de l'infection par un parasite chez le poisson, de préférence un salmonidé, dans laquelle ledit composé est un composé de formule (II) ;
R¹-N=C=S (II)
où R¹ est un alkyle en C₁-C₄ ou un alkényle en C₂-C₃ ou un alkynyle en C₂-C₃ **ou un** phénylalkyle.
